# EUROPEAN PATENT SPECIFICATION

(11) **EP 3 961 171 B1**
(45) Date of publication and mention of the grant of the patent: **18.02.2026**
(21) Application number: 21189131.2
(22) Date of filing: 02.08.2021
(51) Int. Cl.: G01K 13/20, G01K 11/12, G01K 7/42, A61B 5/01, A61B 5/00

(54) **APPARATUS AND METHOD FOR MEASURING BODY TEMPERATURE, AND APPARATUS FOR ESTIMATING BIO-INFORMATION**
VORRICHTUNG UND VERFAHREN ZUR MESSUNG DER KÖRPERTEMPERATUR UND VORRICHTUNG ZUM SCHÄTZEN VON BIOINFORMATIONEN
APPAREIL ET PROCÉDÉ DE MESURE DE LA TEMPÉRATURE CORPORELLE ET APPAREIL D'ESTIMATION D'INFORMATIONS BIOLOGIQUES

(30) Priority: 25.08.2020 KR 20200106958
(43) Date of publication of application: 02.03.2022
(73) Proprietor: Samsung Electronics Co., Ltd., Suwon-si, Gyeonggi-do 16677 (KR)
(72) Inventor: Park, Sang Yun, Gyeonggi-do (KR); Kang, Jae Min, Seoul (KR); Noh, Seung Woo, Gyeonggi-do (KR); Choi, Jin Woo, Gyeonggi-do (KR)
(74) Representative: Grünecker Patent- und Rechtsanwälte PartG mbB

(56) References cited:
- WO-A1-2016/108071
- US-A1- 2011 098 609
- US-A1- 2016 367 149
- US-A1- 2017 095 168
- US-A1- 2017 251 935
- US-A1- 2019 076 032

## Description

### BACKGROUND

### 1. Field

Example embodiments of the disclosure relate to an apparatus and a method for measuring a body temperature of an object using an optical sensor, and an apparatus for estimating bio-information using the body temperature measuring function.

### 2. Description of the Related Art

Generally, body temperature is one of four vital signs and has very important clinical significance. A body temperature sensor may be used in various applications, such as checking infections in patients, thermal side effects of medications, time of ovulation in women, and the like. However, skin temperature may vary depending on external temperature, such that it is difficult to measure deep body temperature by using a portable device such as a wearable device. A general body temperature sensor may be classified into a contact type sensor and a non-contact type sensor. Examples of the contact type sensor may include a sensor for detecting a change in electrical resistance, such as a Resistance Temperature Detector (RTD), a thermistor, etc., a thermocouple for detecting electromotive force, and the like. Further, examples of the non-contact type sensor may include a thermopile, a micro-bolometer, and the like, which measure body temperature by detecting infrared rays radiating from a body surface. A general body temperature measuring technology is greatly affected by a change in environment factors that affect heat transfer, such as a change in external ambient temperature, humidity, air flow, and the like.
US 2011/098609 A1 discloses a hydrogel-containing temperature indicating element for use in ultrasound therapy. The temperature indicating element enable an automated adaptation of the power output as a function of the tissue temperature, avoiding overheating or burning of the skin.
US 2017/095168 A1 discloses a blood pressure measuring apparatus that includes a transparent temperature sensor for measuring the body temperature. The transparent temperature sensor is disposed on an upper portion of a light source and a light receiver to measure the body temperature without blocking light emitted from the light source. Once the temperature of a finger is measured by a temperature sensor, the blood pressure measurer may correct an error of phase difference based on the measured temperature.
WO 2016/108071 A1 discloses a passive planar thermo-optical structure for detection of a region of changed temperature on patient's skin, comprising a thermoactive dye. The thermo-optical structure has a layered structure comprising a transparent film layer, a thermochromic layer, a securing layer, an adhesive layer, and a protective layer. A recording device comprises a central unit, a housing adapted to separate a region of patient's skin from ambient light, a digital camera for acquisition of colours displayed on a thermo-optical structure adhered to patient's skin. A method of diagnosing allergy comprises the steps of stimulation, measurement and comparison. The stimulation step includes exposition of a first region of patient's skin to a histamine solution, exposition of a second region of patient's skin to a neutral solution and exposition of a third region of patient's skin to a test substance. In the measurement step the evaluation of the extent of histamine reaction in the first, the second, and the third region is performed. In the comparison step the extent of histamine reaction in the third region is compared to the extents of histamine reaction in the first region and in the second region. The evaluation of the extent of histamine reaction in the first, the second, and the third region is done based on the extent of the change in colour of the thermo-optical structure, corresponding to the first, the second, and the third region, respectively.
US 2017/251935 A1 discloses a wrist-worn device that comprises an external surface that is not in contact with the user when the wrist-worn device is worn, a force sensor, a PPG sensor disposed on the wrist-worn device, and control logic configured to: (i) generate one or more sensor data samples, each sensor data sample including data that links force data generated by the force sensor when a user presses against the external surface at a given time with heart rate data obtained from the PPG sensor at the given time; and (ii) calculate an estimate of blood pressure from the one or more sensor data samples.

### SUMMARY

It is therefore the object of the present invention to provide an improved method of measuring a body temperature for determining a body temperature of an object by using detected light, and a corresponding apparatus for estimating bio-information.
This object is solved by the subject matter of the independent claims.
Preferred embodiments are defined by the dependent claims.

### BRIEF DESCRIPTION OF THE DRAWINGS

The above and other aspects, features, and advantages of certain embodiments of the disclosure will be more apparent from the following description taken in conjunction with the accompanying drawings.
FIG. 1 is a block diagram illustrating an apparatus for measuring a body temperature according to an example embodiment.
FIGS. 2A, 2B, 2C, 2D, and 2E are diagrams illustrating examples of a thermochromic part according to the example embodiment of FIG. 1.
FIGS. 3A and 3B are diagrams explaining an example of measuring a body temperature according to example embodiments.
FIG. 4 is a block diagram illustrating an apparatus for measuring a body temperature according to an example embodiment.
FIG. 5 is a flowchart illustrating a method of measuring a body temperature according to an example embodiment.
FIG. 6 is a flowchart illustrating a method of measuring a body temperature according to the claimed invention.
FIGS. 7A and 7B are block diagrams illustrating an apparatus for estimating bio-information according to example embodiments.
FIGS. 8A and 8B are diagrams explaining an example of estimating a blood pressure using oscillometry.
FIGS. 9 and 10 are block diagrams illustrating an apparatus for estimating bio-information according to example embodiments.
FIGS. 11, 12, and 13 are diagrams illustrating various structures of an apparatus for estimating bio-information according to example embodiments.

### DETAILED DESCRIPTION

Details of example embodiments are included in the following detailed description and drawings. Advantages and features of the disclosure, and a method of achieving the same will be more clearly understood from the following embodiments described in detail with reference to the accompanying drawings. Throughout the drawings and the detailed description, unless otherwise described, the same drawing reference numerals will be understood to refer to the same elements, features, and structures.

It will be understood that, although the terms first, second, etc. may be used herein to describe various elements, these elements should not be limited by these terms. These terms are only used to distinguish one element from another. Also, the singular forms are intended to include the plural forms as well, unless the context clearly indicates otherwise. It will be further understood that when an element is referred to as "comprising" another element, the element is intended not to exclude one or more other elements, but to further include one or more other elements, unless explicitly described to the contrary. In the following description, terms such as "unit" and "module" indicate a unit for processing at least one function or operation and they may be implemented by using hardware, software, or a combination thereof.

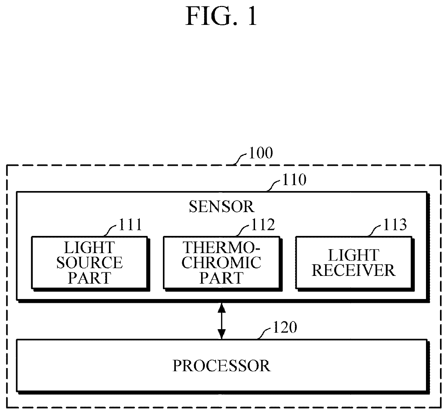

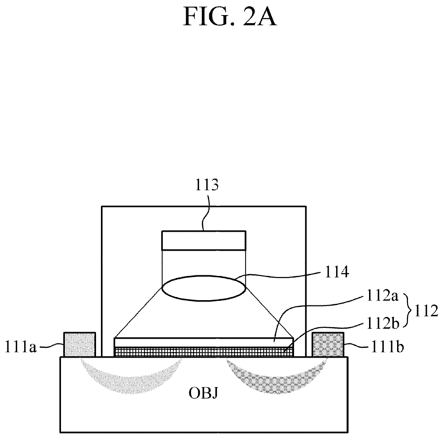

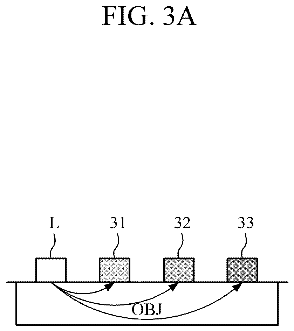

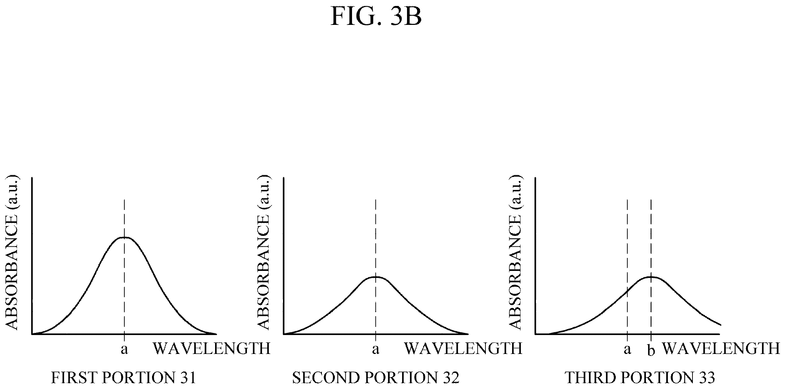

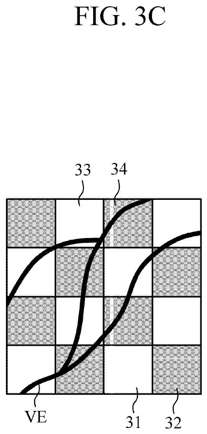

FIG. 1 is a block diagram illustrating an apparatus for measuring a body temperature according to an example embodiment. FIGS. 2A to 2E are diagrams illustrating examples of a thermochromic part according to the example embodiment of FIG. 1. FIGS. 3A and 3B are diagrams explaining an example of measuring a body temperature according to example embodiments.

Referring to FIG. 1, an apparatus 100 for measuring a body temperature includes a sensor 110 and a processor 120.

The sensor 110 is configured to detect a light signal that emanates from an object. For example, the sensor 10 may include a light source part (or a light source) 111 configured to emit light onto the object, and a light receiver 113 configured to detect light scattered or reflected from the object, e.g., a surface of the object and/or a body tissue within the object, such as blood vessels and the like, after the light is emitted onto the object by the light source part 111. Further, the sensor 110 may further include a thermochromic part (or a thermochromic structure) 112 having various thermochromic properties and disposed on a light path in which the light, emitted by the light source part 111, is scattered or reflected from the objects and then travels toward the light receiver 113.

FIG. 2A is a schematic diagram illustrating a structure of the sensor 110 according to an example embodiment.

Referring to FIG. 2A, the light source part 110 may include one or more light sources 111a and 111b. The one or more light sources 111a and 111b may include a light emitting diode (LED), a laser diode (LD), a phosphor, etc., but are not limited thereto. The one or more light sources 111a and 111b may emit light of a relatively long wavelength, so as to detect a light signal at deep parts of an object OBJ. The one or more light sources 111a and 111b may emit light of different wavelengths including, e.g., an infrared wavelength, a green wavelength, a blue wavelength, a red wavelength, and the like. However, the wavelengths are not limited thereto. Although FIG. 2A shows two light sources 111a and 111b, the light source part 110 is not limited thereto and may include one light source or three or more light sources.

The thermochromic part 112 may include a thermally conductive material 112a through which light may be transmitted and heat may be transferred. For example, the thermally conductive material 112a may include a transparent material. Further, the thermally conductive material 112a may have a contact surface which comes into contact with the object OBJ. The thermally conductive material 112a may include a thermochromic liquid crystal, and the thermochromic liquid crystal may include a thermochromic layer 112b having thermochromic properties. Alternatively, the thermochromic layer 112b having thermochromic properties and coated with a thermochromic material, such as a thermochromic pigment, may be formed on a surface of the thermally conductive material 112a.

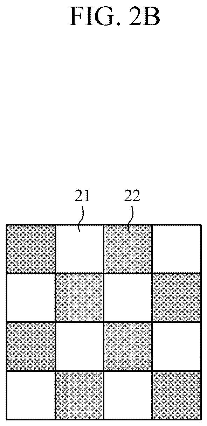

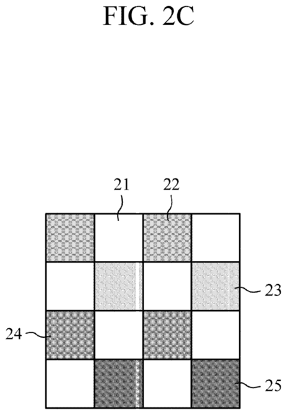

Referring to FIGS. 2B and 2C, the thermochromic layer 112b of the thermochromic part 112 may be formed in a predetermined pattern so as to have various thermochromic properties. For example, as illustrated in FIG. 2B, the thermochromic layer 112b may have portions 21, having no thermochromic properties, and thermochromic portions 22 coated with a thermochromic material so as to react to specific temperatures, and the non-thermochromic portions 21 and the thermochromic portions 22 may be arranged in a predetermined pattern. A shape of the pattern in which the non-thermochromic portions 21 and the thermochromic portions 22 are be arranged is not limited to the example as shown in FIG. 2B. Further, as illustrated in FIG. 2C, the thermochromic layer 112b may have portions 21, having no thermochromic properties, and a plurality of thermochromic portions 22, 23, 24, and 25 that react to different temperatures to change a light spectrum, and the portions 21, 22, 23, 24, and 25 may formed in a pattern.

Referring back to FIG. 2A, the light receiver 113 may detect a light signal which is scattered or reflected from the object OBJ and passes through the thermochromic part 112. According to the pattern of the thermochromic part 112, the light receiver 113 may detect a light spectrum without a thermochromic change and a light spectrum that is changed according to the thermochromic properties of at least a portion of the thermochromic part 112. The light receiver 113 may include, for example, a complementary metal-oxide semiconductor (CMOS) image sensor. However, the light receiver 113 is not limited thereto, and may include a charge-coupled device (CCD) image sensor, a photodiode, a phototransistor, a photodiode array, and the like.

In addition, the sensor 110 may include a light collector 114 that collects light, having passed through the thermochromic part 112, on a light path between the thermochromic part 112 and the light receiver 113. The light collector 114 may include a condensing lens, but is not limited thereto.

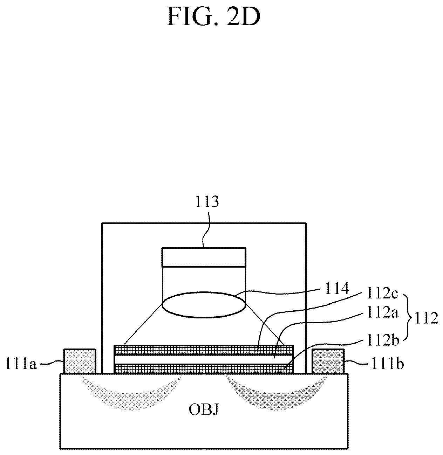

FIG. 2D is a schematic diagram illustrating a structure of the sensor 110 according to another example embodiment.

Referring to FIG. 2D, the thermochromic part 112 may include, for example, the thermally conductive material 112a, and may include a first thermochromic layer 112b, formed on a first surface of the thermally conductive material 112a, and a second thermochromic layer 112c formed on a second surface of the thermally conductive material 112a. If thermal conductivity of the thermally conductive material 112a is too high, it is difficult to calculate a temperature difference by comparing spectra of the first thermochromic layer 112b and the second thermochromic layer 112c; and if thermal conductivity of the thermally conductive material 112a is too low, it is difficult for a heat flux to reach thermal equilibrium, thereby resulting in a delayed measurement response. Accordingly, the sensor 110 may be configured in various manners so that the thermal conductivity of the thermally conductive material 112a may have a proper value. For example, the thickness of the thermally conductive material 112a may be adjusted, an air layer may be formed in the thermally conductive material 112a, or the thermally conductive material 112a may be formed with a porous transparent material.

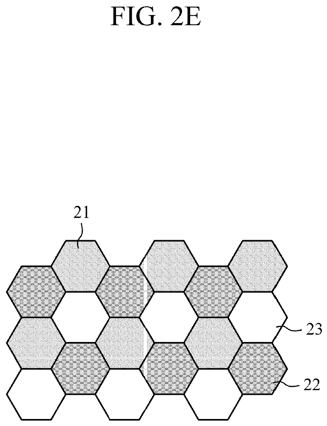

The first thermochromic layer 112b and the second thermochromic layer 112c may be formed in different patterns. FIG. 2E is a diagram illustrating the first thermochromic layer 112b and the second thermochromic layer 112c which are superimposed on each other. As illustrated in FIG. 2E, the first thermochromic layer 112b and the second thermochromic layer 112c, when superimposed on each other, may have portions 21 having thermochromic properties at a first temperature, portions 22 having thermochromic properties at a second temperature, and non-thermochromic portions 23, and the portions 21, 22, and 23 are formed in a pattern. That is, the non-thermochromic portions 23, the portions 21 having thermochromic properties at the first temperature, and the portions 22 having thermochromic properties at the second temperature may be patterned so that spectra of these portions may be measured uniformly. The first temperature and the second temperature may be different from each other, but the first and the second temperatures are not necessarily limited thereto and may be the same temperature.

Referring back to FIG. 1, the processor 120 may be electrically connected to the sensor 110 to control the sensor 110. The processor 120 may receive a light signal from the sensor 110, and may measure body temperature of the object based on the light signal.

For example, the processor 120 may measure body temperature of the object by comparing a light spectrum, changed by the thermochromic part 112, and a reference spectrum. The reference spectrum may be a light spectrum changed according to a light absorption characteristic of a body tissue of the object. For example, if a portion of the thermochromic part 112 has no thermochromic properties, the reference spectrum may be a spectrum of light, having passed through the portion having no thermochromic properties, without undergoing a thermochromic change. In another example, the reference spectrum may be a spectrum previously measured from the object at a calibration time.

Referring to FIG. 3A, for example, (1) of FIG. 3A illustrates a light source L, a first portion 31 having no thermochromic properties of the thermochromic part 112, a second portion 32 reacting to a temperature of 34 °C, and a third portion reacting to a temperature of 35 °C; and (2) of FIG. 3A illustrates a first spectrum of the first portion 31, a second spectrum of the second portion 32, and a third spectrum of the third portion 33, which are detected by the light receiver 113.

For example, the thermochromic part 112 may include only the first portion 31 and the second portion 32 that are arranged in a pattern, and the processor 120 may calculate a similarity between the first spectrum of light that does not undergo a thermochromic change and the second spectrum of light that undergoes a thermochromic change. Based on the similarity being greater than or equal to a predetermined threshold value, the processor 120 may estimate that a body temperature of the object is greater than or equal to 34 °C. As illustrated in FIG. 3A, the first spectrum and the second spectrum have different magnitudes of absorbance but have the same peak wavelength, such that it may be determined that a similarity between the first spectrum and the second spectrum is sufficiently high. Because absorbance of light emanating from the object is changed again according to a light absorption characteristic of the second portion 32, the processor 120 may calculate the similarity after normalizing the magnitude of absorbance of the second portion 32 to correspond to the magnitude of absorbance of the first spectrum.

In another example, as illustrated in FIG. 3A, the thermochromic part 112 may include the first portion 31, the second portion 32, and the third portion 33 that are arranged in a pattern, the processor 120 may calculate a first similarity between the first spectrum that does not undergo the thermochromic change and the second spectrum that undergoes the thermochromic change due to the second portion 32, and a second similarity between the first spectrum that does not undergo the thermochromic change and a third spectrum that undergoes the thermochromic change due to the third portion 33. Based on the first similarity being greater than or equal to a predetermined threshold value, and the second similarity being less than the predetermined threshold value, the processor 120 may determine a temperature value between 34 °C and 35 °C as a body temperature of the object. In an example embodiment, the processor 120 may determine an average temperature value of 34.5 °C as a body temperature of the object. In another example embodiment, the processor 120 may apply different weights to the first similarity and the second similarity, and determine a weighted average as the body temperature of the object.

The similarity may include Euclidean distance, Pearson correlation coefficient, Spearman correlation coefficient, Cosine similarity, and the like.

In yet another example, the processor 120 may input spectra of light, having passed through each portion of the thermochromic part 112, into a pre-generated body temperature measurement model, and may obtain a result of the body temperature measurement model as the body temperature of the object. For example, the light receiver 113 may obtain pattern images of respective portions having different thermochromic properties, and the processor 120 may obtain the body temperature of the object by comparing and analyzing spectra of the respective portions by using a body temperature measurement model. The body temperature measurement model may be pre-generated based on machine learning, such as Neural Network, Deep Neural Network, Convolutional Neural Network, etc., or using artificial intelligence and the like.

Generally, compared to a deep body temperature, the temperature of skin is greatly affected by an external temperature. It has been known that there is a constant correlation between a body temperature and a heart rate, and in order to compensate for the effect of an external temperature, the heart rate may be further considered in measurement of the deep body temperature. To this end, the body temperature measurement model may be pre-generated so that the body temperature of the object may be measured by further considering a user's heart rate, along with light spectra changed by the thermochromic part 112.

In this case, the processor 120 may extract a pulse wave signal based on a light signal detected by the light receiver 113 for a predetermined period of time, and may extract a heart rate based on the extracted pulse wave signal. For example, when a user places an object on the sensor 110 and changes a contact pressure for a predetermined period of time, a pulse wave amplitude is changed, and the processor 120 may extract the pulse wave signal based on the change in the pulse wave amplitude. Further, by applying the body temperature measurement model, the processor 120 may obtain a deep body temperature value based on the heart rate along with the light spectra changed by the thermochromic part 112.

Referring to FIG. 3B, the processor 120 may estimate the presence and position of blood vessels VE within the object based on the spectra of light having passed through the thermochromic part 112 and detected by the light receiver 113. In an example embodiment, the light source part 111 may include a light source that emits light of an infrared wavelength. For example, blood in the blood vessels VE flowing into the tissue has a heat flux, such that it can be generally estimated that a temperature in a blood vessel region is higher than a temperature in other regions. For example, as illustrated in FIG. 3B, a temperature of portions 33 and 34, where blood vessels are located, is relatively higher than a temperature in portions 31 and 32 where no blood vessels are located.

As described above, the processor 120 may determine the body temperature of respective portions of the object based on the patterned images of the thermochromic part 112, and may estimate portions, having a relatively higher body temperature, as portions where blood vessels are located. Further, by comparing a body temperature of a portion, where blood vessels are located, with a portion where no blood vessels are located, or based on the pre-generated body temperature measurement model, the processor 120 may determine a final body temperature of the object.

In addition, if the light source part 111 includes a light source having a uniform characteristic, e.g., a laser light source, imaging of blood vessels may be provided, and the processor 120 may estimate a position of a blood vessel based on blood vessel images in the manner as described above. Further, the processor 120 may quantify blood perfusion based on the blood vessel images, and may obtain a measured body temperature value by using a body temperature measurement model to which the blood perfusion is further applied. For example, the processor 120 may calculate a contrast value from a laser speckle contrast image, and may quantify blood perfusion based on the calculated contrast value. As a technique for quantifying blood perfusion, various known techniques may be used, such that detailed description thereof will be omitted.

In an example embodiment, when the thermochromic part 112 has the thermally conductive material 112a, the first thermochromic layer 112b, and the second thermochromic layer 112c as illustrated in FIG. 2D, the processor 120 may estimate a first body temperature based on a spectrum changed by the first thermochromic layer 112b, and may estimate a second body temperature based on a spectrum changed by the second thermochromic layer 112c. In addition, the processor 120 may estimate a heat flux based on the first temperature, the second temperature, and thermal conductivity of the thermally conductive material 112a.

Furthermore, the processor 120 may obtain a final body temperature of the object based on the obtained first body temperature, second body temperature, and heat flux. For example, the processor 120 may determine a final body temperature of the object by using a body temperature measurement model, which is trained to measure a body temperature by using, as inputs, the first body temperature, the second body temperature, and the heat flux.

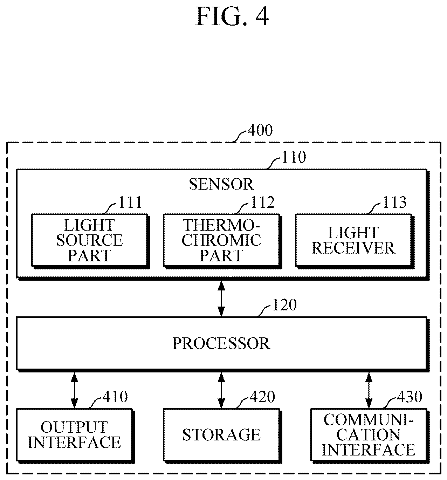

FIG. 4 is a block diagram illustrating an apparatus for measuring a body temperature according to another example embodiment.

Referring to FIG. 4, an apparatus 400 for measuring a body temperature includes the sensor 110, the processor 120, an output interface 410, a storage 420, and a communication interface 430. The sensor 110 includes the light source part (or light source) 111, the thermochromic part (or thermochromic structure) 112, and the light receiver 113. The sensor 110 and the processor 120 are described above in detail and repetitive descriptions will be omitted.

The output interface 410 may provide processing results of the processor 120 to a user. For example, the output interface 410 may display a measured body temperature value of the processor 120 on a display. In this case, if a measured body temperature value falls outside a normal range, the output interface 410 may provide a user with warning information by changing one or more display attributes such as a color, a line thickness, etc., and/or displaying the abnormal value along with a normal range, so that the user may easily recognize the abnormal value. Further, along with or without the visual output, the output interface 410 may output information, related to the measured body temperature value of the user, in a non-visual manner by a voice, a vibration, a tactile sensation, and/or the like by using, for example, a voice output module such as a speaker, a haptic module and/or the like.

The storage 420 may store information related to measuring a body temperature. For example, the storage 420 may store light spectra, obtained by the sensor 110, and data generated by the processor 120 during measurement of a body temperature. Further, the storage 420 may store reference information, such as a reference spectrum related to measuring a body temperature, a body temperature measurement model, and the like. The storage 420 may include at least one storage medium of a flash memory type memory, a hard disk type memory, a multimedia card micro type memory, a card type memory (e.g., an SD memory, an XD memory, etc.), a Random Access Memory (RAM), a Static Random Access Memory (SRAM), a Read Only Memory (ROM), an Electrically Erasable Programmable Read Only Memory (EEPROM), a Programmable Read Only Memory (PROM), a magnetic memory, a magnetic disk, and an optical disk, and the like, but is not limited thereto.

The communication interface 430 may communicate with an external device to transmit and receive various data, related to measuring a body temperature, to and from the external device. The external device may include an information processing device such as a smartphone, a tablet PC, a desktop computer, a laptop computer, and the like. For example, the communication interface 430 may transmit a body temperature measurement result to an external device, such as a user's smartphone and the like, so that the user may manage and monitor the measurement result by using a device having a relatively high performance.

The communication interface 430 may communicate with the external device by using various wired and/or wireless communication techniques, such as Bluetooth communication, Bluetooth Low Energy (BLE) communication, Near Field Communication (NFC), WLAN communication, Zigbee communication, Infrared Data Association (IrDA) communication, Wi-Fi Direct (WFD) communication, Ultra-Wideband (UWB) communication, Ant+ communication, WIFI communication, Radio Frequency Identification (RFID) communication, 3G communication, 4G communication, 5G communication, and the like. However, these are merely examples and are not intended to be limiting.

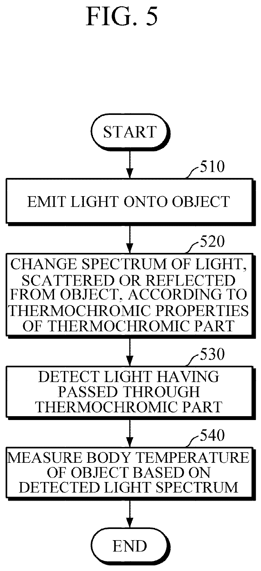

FIG. 5 is a flowchart illustrating a method of measuring a body temperature according to an example embodiment.

The method of FIG. 5 is an example of a method of measuring a body temperature which may be performed by any one of the apparatuses 100 and 400 for measuring a body temperature according to the example embodiments of FIGS. 1 and 4, which are described above in detail and thus will be briefly described below in order to avoid redundancy.

The apparatuses 100 and 400 for measuring a body temperature may emit light onto an object by a light source part in 510. The light source part may emit light of one or more wavelengths including, for example, an infrared wavelength, a green wavelength, a blue wavelength, a red wavelength, and the like.

The light, emitted by the light source part onto the object, is scattered or reflected from the surface of the object or from a body tissue within the object, and passes through the thermochromic part of the sensor such that a spectrum of the light is changed according to thermochromic properties of the thermochromic part in 520. The thermochromic part may have one or more portions, having different thermochromic properties, which are patterned in the thermochromic part by using a thermochromic pigment, a thermochromic liquid crystal, or the like. In an example embodiment, the thermochromic part may have one or more portions having different thermochromic properties and portions having no thermochromic properties.

Subsequently, the apparatuses 100 and 400 for measuring a body temperature may detect, by the light receiver, light scattered or reflected from the object and having passed through the thermochromic part in 530. The light receiver may detect one or more light spectra changed according to thermochromic properties of the thermochromic part. For example, the light spectra may include a light spectrum, having passed through portions having no thermochromic properties, without a thermochromic change, and a light spectrum, having passed through portions having different thermochromic properties, and in which a thermochromic change has occurred.

Next, the processor of the apparatuses 100 and 400 for measuring a body temperature may obtain a measured body temperature value of the object based on the light spectrum detected by the light receiver in 540. For example, the apparatuses 100 and 400 for measuring a body temperature may determine a measured body temperature value by comparing the spectrum, having passed through portions having thermochromic properties of the thermochromic part, in which the thermochromic change has occurred, with a reference spectrum in which the thermochromic change has not occurred. In an example embodiment, the reference spectrum may be a spectrum having passed through portions having no thermochromic properties of the thermochromic part. In this case, the apparatuses 100 and 400 for measuring a body temperature may obtain the measured body temperature value by comparing a similarity between one or more spectra in which the thermochromic change has occurred and the reference spectrum, or by using a pre-defined body temperature measurement model. **In** addition, the apparatuses 100 and 400 for measuring a body temperature may estimate a blood vessel position based on the light spectra and may estimate a body temperature at the blood vessel position and a body temperature at positions other than the blood vessel position, and may determine the body temperature of the object based on the estimated body temperatures.

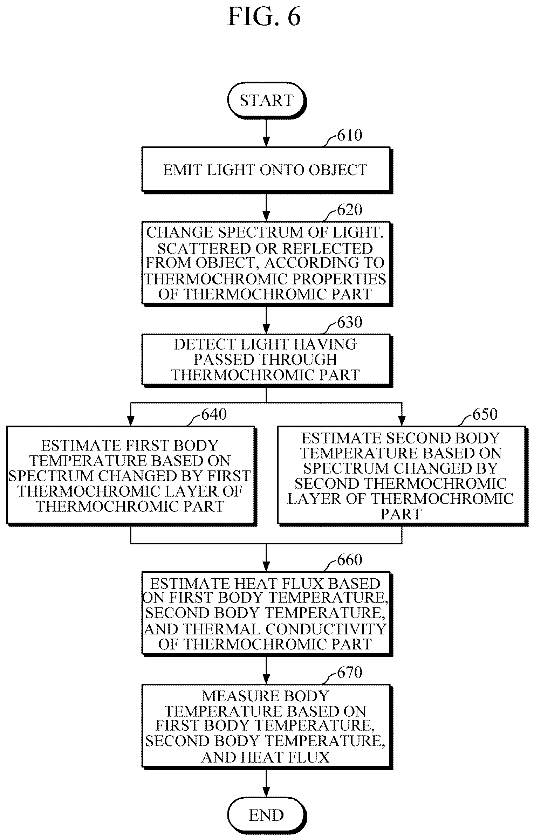

FIG. 6 is a flowchart illustrating a method of measuring a body temperature according to the claimed invention.

The method of FIG. 6 is an example of a method of measuring a body temperature which may be performed by any one of the apparatuses 100 and 400 for measuring a body temperature according to the example embodiments of FIGS. 1 and 4, which are described above in detail and thus will be briefly described below in order to avoid redundancy.

The apparatuses 100 and 400 for measuring a body temperature emit light onto an object by a light source part in 610. The light source part may emit light of one or more wavelengths including, for example, an infrared wavelength, a green wavelength, a blue wavelength, a red wavelength, and the like.

The light, emitted by the light source onto the object, is scattered or reflected from the surface of the object or from a body tissue within the object, and passes through the thermochromic part such that a spectrum of the light is changed according to thermochromic properties of the thermochromic part in 620, and the apparatuses 100 and 400 for measuring a body temperature detect, by the light receiver, light scattered or reflected from the object and having passed through the thermochromic part in 630. In this case, the thermochromic part may have a first thermochromic layer and a second thermochromic layer which are formed in a pattern on each of both surfaces of a thermally conductive material.

Subsequently, the apparatuses 100 and 400 for measuring a body temperature estimate a first body temperature based on the spectrum changed by the first thermochromic layer of the thermochromic part in 640, and estimate a second body temperature based on the spectrum changed by the second thermochromic layer of the thermochromic part in 650.

Next, the apparatuses 100 and 400 for measuring a body temperature estimate a heat flux based on the first body temperature, the second body temperature, and thermal conductivity of the thermochromic part in 660.

Then, the apparatuses 100 and 400 for measuring a body temperature obtain a measured body temperature value based on the first body temperature, the second body temperature, and the heat flux by using a pre-defined body temperature model in 670.

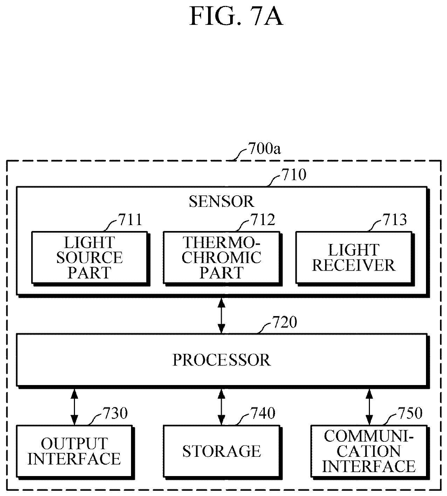

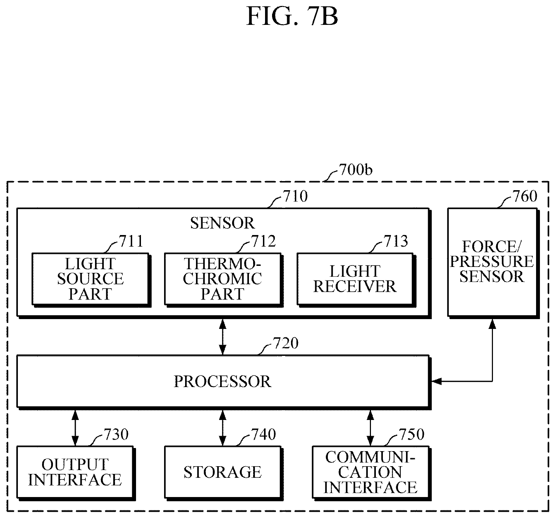

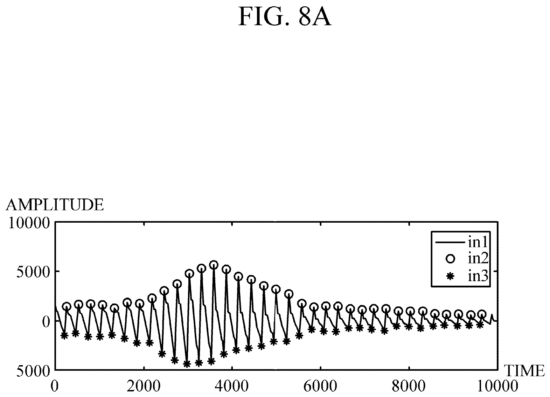

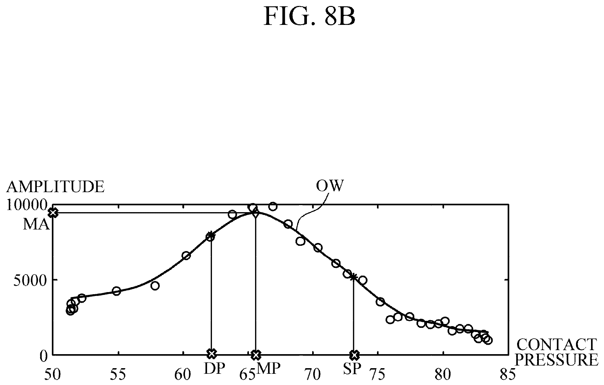

FIGS. 7A and 7B are block diagrams illustrating an apparatus for estimating bio-information according to example embodiments. FIGS. 8A and 8B are diagrams explaining an example of estimating a blood pressure using oscillometry.

Hereinafter, for ease of description, the apparatus for estimating bio-information may also be referred to as a healthcare device. Various examples of the healthcare device, which will be described below, may include electronic devices such as a smart watch, a smart band, smart glasses, smart earphones, a smart ring, a smart necklace, a smartphone, a tablet PC, etc., and may perform only the aforesaid function of measuring a body temperature, or may perform the function of estimating bio-information along with the body temperature measuring function. The bio-information that may be estimated by the healthcare device may include at least one among triglyceride, body fat percentage, body water, blood glucose, cholesterol, carotenoid, protein, uric acid, blood pressure, vascular age, arterial stiffness, aortic pressure waveform, vascular compliance, stress index, fatigue level, skin age, skin elasticity, and the like, but is not limited thereto. For convenience of explanation, the following description will be given using a blood pressure as an example.

Referring to FIGS. 7A and 7B, the healthcare devices 700a and 700b according to the example embodiments may include a sensor 710, a processor 720, an output interface 730, a storage 740, and a communication interface 750. In addition, the healthcare device 700b of FIG. 7B may further include a force/pressure sensor 760. The force/pressure sensor 760 may be a single force sensor, a force sensor array, a combination of a force sensor and an area sensor, and the like. The force sensor may include a strain gauge, but is not limited thereto.

The sensor 710 may include a light source part (or light source) 711, a thermochromic part (or thermochromic structure) 712, and a light receiver 713. The sensor 710 may be disposed on a surface which comes into contact with an object when the healthcare devices 700a and 700b are worn on the object. The sensor 710 may detect a light signal when the object, being in contact with the sensor 710, changes a contact pressure for a predetermined period of time to induce a change in a pulse wave amplitude, as described above. The light source 711 may include a light source, such as an LED, an LD, a phosphor, etc., which emits light of one or more wavelengths. A spectrum of light, scattered or reflected from the object, is changed by the thermochromic part 712 having various thermochromic properties, and the light receiver 714 may detect light spectra corresponding to the respective portions of the thermochromic part 712.

The processor 720 may measure a body temperature based on the light spectra changed by the thermochromic part 712 and detected by the sensor 710, as described above. Various above embodiments performed by the apparatuses 100 and 400 for measuring a body temperature may be performed by the processor 720 according to an example embodiment, such that detailed description thereof will be omitted.

In addition, upon estimating a blood vessel position based on the light spectra detected by the sensor 710 as described above, the processor 720 may guide a user on the contact of the object based on the estimated blood vessel position. For example, if there is no estimated blood vessel position or if the blood vessel position is not within a pre-defined range in a detection area of the light receiver 713, the processor 720 may guide a user so that the object may come into contact again with the sensor 710.

Further, the processor 720 may extract a pulse wave signal based on an intensity of the light signal detected by the sensor 710 for a predetermined period of time. For example, the processor 720 may extract the pulse wave signal based on a signal of light having passed through portions having no thermochromic properties of the thermochromic part 712. Alternatively, the processor 720 may extract the pulse wave signal based on a signal of light corresponding to the estimated blood vessel position. However, the pulse wave signal is not limited thereto.

The processor 720 may obtain a contact pressure based on the intensity of the light signal detected for a predetermined period of time. For example, if the object increases a pressing force on the sensor 710, a contact area is generally increased, such that a quantity of light detected by the light receiver 714 is increased. Accordingly, by using a contact pressure conversion model which defines a correlation between the detected quantity of light and the contact pressure, the processor 720 may convert the intensity of the light signal, detected by the light receiver 713, into a contact pressure. In another example, as illustrated in FIG. 7B, if the healthcare device 700b includes the force/pressure sensor 760, the force/pressure sensor 760 may obtain a contact force and/or contact pressure when the object, being in contact with the sensor 710, changes the contact force. Upon obtaining the contact force, the force/pressure sensor 760 may obtain a contact pressure by using an area of the sensor 710.

Once the pulse wave signal and the contact pressure are obtained, the processor 720 may estimate a blood pressure using oscillometry.

For example, FIG. 8A illustrates a change in an amplitude of a pulse wave signal when the object, being in contact with the sensor 710, gradually increases a contact pressure. FIG. 8B illustrates an oscillogram OW representing a relationship between a change in a contact pressure and the amplitude of a pulse wave signal.

The processor 720 may extract, e.g., a peak-to-peak point of the pulse wave signal waveform by subtracting a negative (-) amplitude value in3 from a positive (+) amplitude value in2 of a waveform envelope in1 at each measurement time of the obtained pulse wave signal, and may obtain the oscillogram OW by plotting the peak-to-peak amplitude at each measurement time against the contact pressure value at a corresponding time and by performing, for example, polynomial curve fitting.

The processor 720 may estimate a blood pressure by using the generated oscillogram OW. For example, the processor 720 may estimate a mean arterial pressure (MAP) based on a contact pressure value MP at a maximum point MA of the pulse wave in the oscillogram. For example, the processor 720 may determine, as MAP, the contact pressure value MP itself at the maximum point MA of the pulse wave. Alternatively, the processor 720 may estimate a MAP by applying the contact pressure value MP to a pre-defined MAP estimation equation. In this case, the MAP estimation equation may be expressed in the form of various linear or non-linear combination functions, such as addition, subtraction, division, multiplication, logarithmic value, regression equation, and the like, with no particular limitation.

Further, the processor 720 may estimate a diastolic blood pressure (DBP) and a systolic blood pressure (SBP) based on contact pressure values DP and SP at points to the left and to the right of an amplitude value at the maximum point MA of the pulse wave and having a preset ratio, e.g., 0.5 to 0.7, to the amplitude value at the maximum point MA. Likewise, the processor 720 may also determine the contact pressure values DP and SP as a DBP and a SBP, respectively. Further, the processor 720 may estimate DBP and SBP by using pre-defined DBP estimation equation and SBP estimation equation.

In addition, the processor 720 may operate in a first mode for measuring a body temperature, in a second mode for measuring a blood pressure, and in a third mode for measuring a both body temperature and a blood pressure. Any one of the first mode, the second mode, and the third mode may be set as a default mode, and may be changed by a user.

The output interface 730 may provide processing results of the processor 720. For example, the output interface 730 may display a measured body temperature value and/or an estimated blood pressure value of the processor 120 on a display. In this case, the output interface 730 may display warning information to a user based on whether the measured body temperature value or the estimated blood pressure value falls outside a normal range, in which the output interface 730 may provide a user with the warning information in a non-visual manner by voice, vibrations, tactile sensation, and the like using a voice output module such as a speaker, or a haptic module and the like.

The storage 740 may store a variety of information related to measuring a body temperature and/or estimating blood pressure. For example, the storage 740 may store the light signal obtained by the sensor 710, the pulse wave signal, the measured body temperature value and/or the estimated blood pressure value which are extracted by the processor 720. In addition, the storage 740 may store a reference spectrum related to measuring a body temperature, a body temperature measurement model, and a variety of information related to estimating blood pressure.

The communication interface 750 may communicate with an external device to transmit and receive various data related to measuring a body temperature and/or estimating blood pressure. In this case, the external device may include an information processing device such as a smartphone, a tablet PC, a desktop computer, a laptop computer, and the like. For example, the communication interface 750 may transmit the measured body temperature value and/or the estimated blood pressure value to an external device, such as a user's smartphone and the like, so that the user may manage and monitor the values by using a device having a relatively high performance.

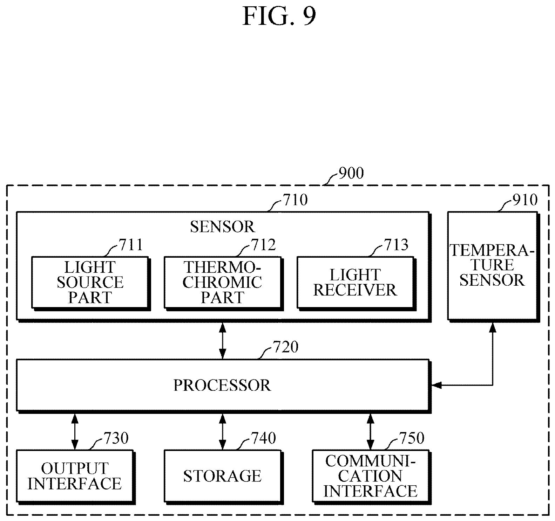

FIG. 9 is a block diagram illustrating a healthcare device according to another example embodiment.

Referring to FIG. 9, a healthcare device 900 includes the sensor 710, the processor 720, the output interface 730, the storage 740, the communication interface 750, and a temperature sensor 910. The sensor 710, the processor 720, the output interface 730, the storage 740, and the communication interface 750 are described above with reference to FIGS. 7A and 7B, such that a redundant description thereof will be omitted. Although not shown in FIG. 9, the healthcare device 900 may further include the force/pressure sensor 760.

The temperature sensor 910 may be disposed on a surface exposed to the outside of the healthcare device 900 and not being in contact with an object when the healthcare device 900 is worn on the object. The temperature sensor 910 may measure an external temperature while the sensor 710 detects a light signal from the object.

As described above, the processor 720 may measure a body temperature based on a light spectrum measured by the sensor 710, and may compensate for the body temperature of the object based on the external temperature measured by the temperature sensor 910. To this end, a body temperature measurement model may be pre-generated, which is trained based on machine learning, artificial intelligence, etc., so that deep body temperature of the object may be measured based on the measured body temperature and external temperature.

The output interface 730 may output the external temperature and the body temperature which are obtained by the processor 720. The storage 740 may store processing results of the processor 720. Further, the communication interface 750 may transmit the external temperature and a body temperature measurement result to an external device.

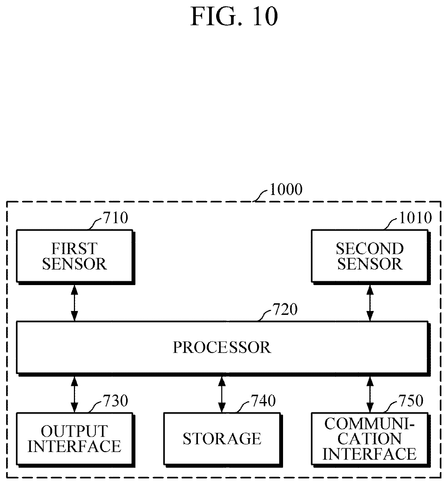

FIG. 10 is a block diagram illustrating a healthcare device according to yet another example embodiment.

Referring to FIG. 10, a healthcare device 1000 includes a first sensor 710, the output interface 730, the storage 740, and the communication interface 750. The healthcare device 1000 may further include a second sensor 1010. The first sensor 710 may be the same as the sensor 710 according to the aforesaid embodiments, and may include the light source part 711, the thermochromic part 712, and the light receiver 713. Further, similarly to the first sensor 710, the second sensor 1010 may include a light source part, a thermochromic part, and a light receiver. The light source part of the second sensor 1010 may use external light as a light source, without having a separate light source.

The first sensor 710 may be disposed on a surface which comes into contact with an object when the healthcare device 1000 is worn on the object. By contrast, the second sensor 1010 may be disposed on another surface being in contact with the object when the healthcare device 1000 is worn on the object, or may be disposed on a surface exposed to the outside and not being in contact with the object.

The processor 720 may measure a body temperature of the object based on a light spectrum detected by the first sensor 710, and in a case where the second sensor 1010 is disposed on the surface exposed to the outside, the processor 720 may measure an external temperature based on a light spectrum detected by the second sensor 1010. As described above, based on measurement of the body temperature of the object and the external temperature, the processor 720 may obtain a final body temperature, which is corrected based on the external temperature, by using a body temperature measurement model.

In addition, in a case where a first portion of the object is in contact with the first sensor 710 and a second portion of the object is in contact with the second sensor 1010 when the healthcare device 1000 is worn on the object, the processor 720 may measure a first body temperature of the first portion of the object and a second body temperature of the second portion of the object, and may determine a user's final body temperature based on at least one of the first body temperature and the second body temperature. For example, the processor 720 may determine the user's final body temperature by selecting any one of the first body temperature and the second body temperature or by combining the first body temperature and the second body temperature. Alternatively, the processor 720 may obtain the user's body temperature by using a body temperature measurement model, in which a final body temperature is output by using the first body temperature and the second body temperature as an input.

Further, the processor 720 may extract a first pulse wave signal of the light detected by the first sensor 710 from the first portion of the object for a predetermined period of time, and based on the second portion of the object that comes into contact with the second sensor 1010, the processor 720 may extract a second pulse wave signal based on a light signal detected from the second portion of the object. The processor 720 may estimate a blood pressure by using the first pulse wave signal and the second pulse wave signal.

For example, the processor 720 may generate a first oscillogram and a second oscillogram based on the first pulse wave signal and the second pulse wave signal, respectively, and may obtain a final estimated blood pressure value by estimating blood pressure values from each of the first oscillogram and the second oscillogram, and combining the estimated blood pressure values.

In another example, the processor 720 may obtain a third pulse wave signal based on the first pulse wave signal and the second pulse wave signal, and may estimate a blood pressure based on the obtained third pulse wave signal using oscillometry. For example, the processor 720 may obtain the third pulse wave signal by subtracting the second pulse wave signal from the first pulse wave signal, but the third pulse wave signal is not limited thereto. Alternatively, the processor 720 may generate a third oscillogram by combining the first oscillogram and the second oscillogram (e.g., subtracting the second oscillogram from the first oscillogram), and may estimate a blood pressure by using the generated third oscillogram. However, the estimation of blood pressure is not limited thereto.

In yet another example, the processor 720 may extract characteristic points from the first pulse wave signal and the second pulse wave signal, may calculate a pulse transit time (PTT) by calculating a time delay between the extracted characteristic points, and may estimate a blood pressure by using the calculated PTT. In this case, the characteristic point may be a maximum point of the pulse wave, but is not limited thereto.

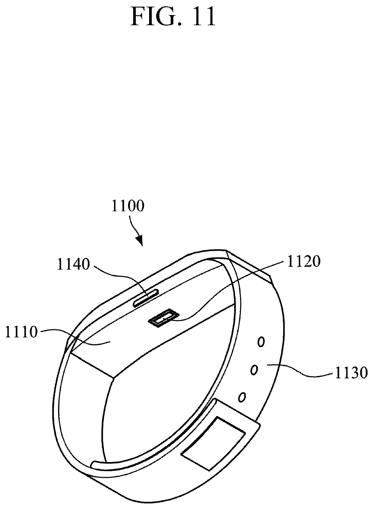

FIGS. 11 to 13 are diagrams illustrating various structures of a healthcare device according to example embodiments. The aforementioned healthcare devices 700a, 700b, 900, and 1000 may be any one of the electronic devices illustrated in FIGS. 11 to 13. Alternatively, some components of the healthcare devices 700a, 700b, 900, and 1000 may be mounted separately in two or more different electronic devices, so that the two or more electronic devices may cooperate to provide a healthcare function. However, examples of the electronic devices are not limited thereto.

For example, referring to FIG. 11, a smart watch type wearable device 1000 includes a main body 1110 and a strap 1130. The main body 1110 may be formed in various shapes, and a battery may be embedded in the main body 1110 and/or the strap 1130 to supply power to various components of the wearable device 1000. The strap 1130 may be flexible so as to be wrapped around a user's wrist. The strap 1130 may include a first strap and a second strap which are separated from each other. A first end of the first strap and a first end the second strap may be connected to both sides of the main body 1110, and a second end of the first strap and a second end of the second strap may be connected to each other via a connecting component. The connecting component may be based on magnetic connection, Velcro connection, pin connection, and the like, but is not limited thereto. Further, the strap 830 is not limited thereto, and may be integrally formed as a non-detachable band.

A sensor 1120 may be disposed on a rear surface of the main body 1110 which comes into contact with the wrist when the main body 1110 is worn on the wrist. The sensor 1120 may include a light source part, a thermochromic part, and a light receiver.

Further, as illustrated in FIG. 7B, a force/pressure sensor may be mounted in the main body 1110 so as to measure a contact force/pressure when the object, being in contact with the sensor 1120, changes a contact pressure. In addition, as illustrated in FIG. 9, a temperature sensor for measuring an external temperature may be disposed on the strap 1130 or a surface of the main body 1110 (e.g., a front surface or a side surface of the main body 1110) which is exposed to the outside when the main body 1110 is worn on the wrist. For example, the temperature sensor may be mounted at a manipulator 1140 disposed on the side surface of the main body 1110.

Moreover, as illustrated in FIG. 10, a second sensor may be disposed on surface of the main body 1110 (e.g., the front surface or the side surface of the main body 1110). For example, the second sensor may be provided on a button of the manipulator 1140 disposed on the side surface of the main body 1110. In this case, the second sensor may include a light source part, a thermochromic part, and a light receiver, in which the light source part may use external light as a light source. As described above, when the main body 1110 is worn on the user's wrist, the second sensor may measure an external temperature while the first sensor 1120 measures a light signal from the wrist, and when the user places another finger on the second sensor, the second sensor may obtain a light signal from the finger.

A processor may be mounted in the main body 1110 and may measure a body temperature and/or estimate a blood pressure based on the light signal detected by the sensor 1120, as described above. The processor may measure a body temperature and/or estimate a blood pressure by further considering additional information measured by the force/pressure sensor, the temperature sensor, and/or the second sensor as described above, thereby improving accuracy in measuring a body temperature and/or estimating a blood pressure.

An output interface may have a display disposed on the front surface of the main body 1110. The display may have a touch screen to receive a touch input. The output interface may be controlled by the processor to display a variety of information. In addition, the output interface may further include an output module, such as a speaker, a haptic module using vibrations, and the like, which are mounted in the main body 1110.

A storage is mounted in the main body 1110, and may store a variety of information processed by the processor or information to be used for measuring a body temperature and/or estimating a blood pressure.

A communication interface is mounted in the main body 1110, and may communicate with an external device to transmit and receive a variety of information required for measuring a body temperature and/or estimating a blood pressure.

The manipulator 1140 may be formed on the side surface of the main body 1110. The manipulator 1140 may receive a user's command and may transmit the received command to the processor. In addition, the manipulator 1140 may have a power button to turn on/off the wearable device 1100.

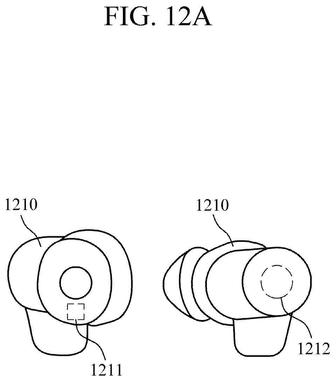

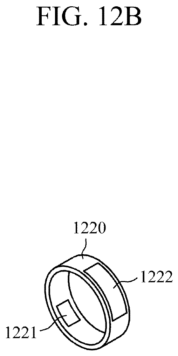

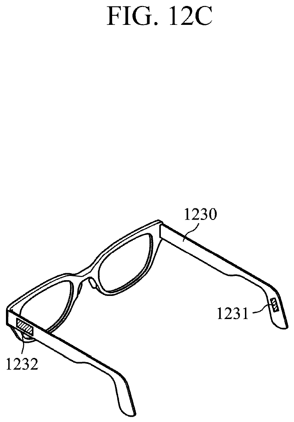

Referring to FIG. 12, the healthcare device may be manufactured as smart earphones, as shown in (1) of FIG. 12, a smart ring, as shown in (2) of FIG. 12, smart glasses, as shown in (3) of FIG. 12, or the like. As illustrated in FIG. 12, when main bodies 1210, 1220, and 1230 of the smart earphones, the smart ring, and the smart glasses are respectively worn on a user's ears, a finger, and a face, first sensors 1211, 1221, and 1231 thereof may be disposed on portions which come into contact with the user's skin. Further, when the main bodies 1210, 1220, and 1230 of the smart earphones, the smart ring, and the smart glasses are worn on the user, second sensors 1212, 1222, and 1232 may be disposed on portions which do not come into contact with the user's skin. In this case, a temperature sensor may be disposed at the positions of the second sensors 1212, 1222, and 1232, or may be disposed at other positions separately from the second sensors 1212, 1222, and 1232.

In addition, a processor and/or a communication interface may be mounted in the main bodies 1210, 1220, and 1230 of the smart earphones 1, the smart ring 2, and the smart glasses 3. Alternatively, the processor may measure a body temperature and/or estimate a blood pressure, and may transmit a measurement result and/or estimation result to an external device through the communication interface. Alternatively, considering the size and the characteristic of a form factor, only the communication interface may be mounted in the main bodies 1210, 1220, and 1230 of the smart earphones, the smart ring, and the smart glasses. The processor may transmit data, measured by the first sensors 1211, 1221, and 1231 and/or the second sensors 1212, 1222, and 1232, and/or the temperature sensor, to the external device through the communication interface, so that a processor of the external device may measure a body temperature and/or estimate a blood pressure, and may provide the processing result to the user through an output interface of the external device.

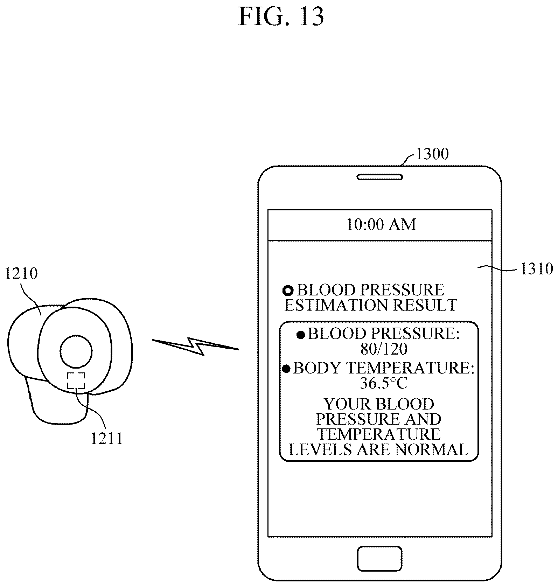

For example, referring to FIG. 13, a healthcare function may be provided in association with the smart earphones and the smartphone. However, this is merely an example, and other electronic devices may cooperate in various manners to provide a healthcare function. For example, a processor for measuring a body temperature and/or estimating a blood pressure may be mounted in a main body 1300 of the smartphone. Upon receiving a request for measuring a body temperature and/or estimating a blood pressure, a processor of the smartphone may communicate with a communication interface of the earphone main body 1210 to obtain data such as a light signal, an external temperature, and the like. Further, upon receiving the data, including the light signal, an external temperature, etc., from the earphones, the processor may measure a body temperature and/or estimate a blood pressure, and may output the processing result on a display through an output interface as illustrated in FIG. 13.

In addition, a sensor for measuring a light signal from a user's finger may be disposed on a rear surface of the main body 1300 of the smartphone, and the second sensor, the temperature sensor, and the like may be disposed at positions of a fingerprint sensor on the front surface of the main body 1300, a power button or a volume button on the side surface of the main body 1300, or at other positions on a surface (e.g., the front and/or rear surface) of the main body 1300, such that a body temperature may be measured and/or a blood pressure may be estimated using only the smartphone. The result of the body temperature measurement and/or blood pressure estimation may be provided on a display 1310 of the smartphone.

Example embodiments of the disclosure may be implemented as a computer-readable code written on a computer-readable recording medium. The computer-readable recording medium may be any type of a recording device in which data is stored in a computer-readable manner.

Examples of the computer-readable recording medium include a ROM, a RAM, a CD-ROM, a magnetic tape, a floppy disc, an optical data storage, and a carrier wave (e.g., data transmission through the Internet). The computer-readable recording medium may be distributed over a plurality of computer systems connected to a network so that a computer-readable code is written thereto and executed therefrom in a decentralized manner. Functional programs, codes, and code segments for implementing the example embodiments of the disclosure may be readily deduced by programmers of ordinary skill in the art to which the disclosure pertains.

At least one of the components, elements, modules or units described herein may be embodied as various numbers of hardware, software and/or firmware structures that execute respective functions described above, according to an example embodiment. For example, at least one of these components, elements or units may use a direct circuit structure, such as a memory, a processor, a logic circuit, a look-up table, etc. that may execute the respective functions through controls of one or more microprocessors or other control apparatuses. Also, at least one of these components, elements or units may be specifically embodied by a module, a program, or a part of code, which contains one or more executable instructions for performing specified logic functions, and executed by one or more microprocessors or other control apparatuses. Also, at least one of these components, elements or units may further include or implemented by a processor such as a central processing unit (CPU) that performs the respective functions, a microprocessor, or the like. Two or more of these components, elements or units may be combined into one single component, element or unit which performs all operations or functions of the combined two or more components, elements of units. Also, at least part of functions of at least one of these components, elements or units may be performed by another of these components, element or units. Further, although a bus is not illustrated in the block diagrams, communication between the components, elements or units may be performed through the bus. Functional aspects of the above embodiments may be implemented in algorithms that execute on one or more processors. Furthermore, the components, elements or units represented by a block or processing operations may employ any number of related art techniques for electronics configuration, signal processing and/or control, data processing and the like.

## Claims

1. A method (500) of measuring a body temperature, the method comprising:
emitting (510) a light onto an object (OBJ) by a light source part (111; 711);
changing (520), by using a thermochromic part (112; 712) , a spectrum of the light, which is reflected or scattered from the object and passes therethrough, according to a thermochromic property of the thermochromic part;
detecting (530) the light having passed through the thermochromic part; and
determining (540) a body temperature of the object by using the spectrum of the detected light, wherein the determining comprises:
estimating a position of a blood vessel (VE) based on the spectrum of the detected light, and
estimating a first body temperature at the estimated position of the blood vessel, estimating a second body temperature at a position other than the position of the blood vessel, and determining the body temperature of the object based on the estimated first body temperature and the estimated second body temperature.

2. The method (500) of claim 1, wherein the determining (540) comprises determining the body temperature of the object (OBJ) by comparing a reference spectrum with the spectrum having changed according to the thermochromic property of the thermochromic part (112; 712), and
wherein the determining comprises determining a spectrum of light, having passed through at least one portion (21) of the thermochromic part having no thermochromic property, as the reference spectrum.

3. The method (500) of claim 1 or 2, further comprising:
estimating a first body temperature based on a spectrum changed by a first thermochromic layer (112b) of the thermochromic part (112; 712);
estimating a second body temperature based on a spectrum changed by a second thermochromic layer (112c) of the thermochromic part; and
estimating a heat flux based on the estimated first body temperature, the estimated second body temperature, and a thermal conductivity of a thermally conductive material (112a) of the thermochromic part, and
wherein the determining (540) comprises determining the body temperature of the object (OBJ) based on the estimated first body temperature, the estimated second body temperature, and the estimated heat flux.

4. An apparatus (100; 700a; 700b; 900; 1000) for estimating bio-information, the apparatus comprising:
a first sensor (110; 710) comprising:
a first light source part (111; 711) configured to emit (510) a first light onto an object (OBJ);
a first thermochromic part (112; 712), configured to change (520) a spectrum of the first light, which is reflected or scattered from the object and passes therethrough, according to a thermochromic property of the first thermochromic part; and
a first light receiver (113; 713) configured to detect (530) the first light having passed through the first thermochromic part; and
a processor (120; 720) configured to determine (540) a body temperature of the object based on the spectrum of the first light detected by the first sensor by estimating a position of a blood vessel (VE) based on the spectrum of the detected light, estimating a first body temperature at the estimated position of the blood vessel, estimating a second body temperature at a position other than the position of the blood vessel, and determining the body temperature of the object based on the estimated first body temperature and the estimated second body temperature.

5. The apparatus (100; 700a; 700b; 900; 1000) of claim 4, wherein the apparatus is at least one of a smart watch, a smart band, smart glasses, smart earphones, a smart necklace, a smartphone, or a tablet personal computer, PC.

6. The apparatus (100; 700a; 700b; 900; 1000) of claim 5, further comprising a force/pressure sensor (760) configured to obtain a contact force and/or a contact pressure between the first sensor (110; 710) and the object (OBJ) that comes into contact with the first sensor, and wherein the processor (120; 720) is further configured to generate an oscillogram based on a pulse wave signal and the contact force and/or the contact pressure, and estimate bio-information based on the generated oscillogram, wherein the pulse wave signal is extracted by the processor based on an intensity of the first light being detected for a predetermined period of time.

7. The apparatus (100; 700a; 700b; 900; 1000) of claim 5 or 6, further comprising a second sensor (1010), the second sensor comprising a second light source part configured to emit a second light onto the object (OBJ), a second thermochromic part configured to change a spectrum of the second light, which is reflected or scattered from the object and passes therethrough, according to a thermochromic property of the second thermochromic part, and a second light receiver configured to detect the second light having passed through the second thermochromic part.

8. The apparatus (100; 700a; 700b; 900; 1000) of claim 7, wherein the processor (120; 720) is further configured to estimate the bio-information based on a first pulse wave signal, obtained when a first object comes into contact with the first sensor, and a second pulse wave signal obtained when a second object comes into contact with the second sensor.

9. The apparatus (100; 700a; 700b; 900; 1000) of claim 8, wherein the processor (120; 720) is further configured to obtain a pulse transit time, PTT, based on the first pulse wave signal and the second pulse wave signal, and estimate the bio-information based on the obtained PTT, or
wherein the processor is further configured to obtain a third pulse wave signal based on the first pulse wave signal and the second pulse wave signal, and estimate the bio-information based on the obtained third pulse wave signal using oscillometry.

10. The apparatus (100; 700a; 700b; 900; 1000) of claim 7, wherein:
the first sensor (110; 710) is disposed on a first surface of a main body of the apparatus which comes into contact with the object (OBJ); and
the second sensor (1010) is disposed on a second surface of the main body, the second surface being exposed to an outside when the object is in contact with the first sensor.

11. The apparatus (100; 700a; 700b; 900; 1000) of claim 10, wherein the processor (120; 720) is further configured to determine the body temperature of the object (OBJ) based on the spectrum of the first light detected by the first light receiver (113; 713), obtain an external temperature based on the spectrum of the second light detected by the second light receiver, and correct the determined body temperature of the object based on the obtained external temperature.

12. The apparatus (100; 700a; 700b; 900; 1000) of claim 7, wherein the second light source part of the second sensor (1010) is based on an external light of the apparatus.

13. The apparatus (100; 700a; 700b; 900; 1000) of claim 5, further comprising a temperature sensor disposed on a surface of a main body of the apparatus, the surface being exposed to an outside when the object is in contact with the first sensor,
wherein the processor (120; 720) is further configured to determine the body temperature of the object (OBJ) based on the spectrum of the first light detected by the first light receiver (113; 713), and correct the determined body temperature of the object based on an external temperature obtained by the temperature sensor.

14. The apparatus (100; 700a; 700b; 900; 1000) of claim 6, wherein the bio-information comprises at least one of triglyceride, body fat percentage, body water, blood glucose, cholesterol, carotenoid, protein, uric acid, blood pressure, vascular age, arterial stiffness, aortic pressure waveform, vascular compliance, stress index, fatigue level, skin age, or skin elasticity.

## Patentansprüche

1. Verfahren (500) zum Messen einer Körpertemperatur, wobei das Verfahren umfasst:
Ausstrahlen (510) von Licht auf ein Objekt (OBJ) durch einen Lichtquellenteil (111; 711);
Ändern (520) eines Spektrums des Lichts, das vom Objekt reflektiert oder gestreut wird und dieses passiert, unter Verwendung eines thermochromen Teils (112; 712) entsprechend einer thermochromen Eigenschaft des thermochromen Teils;
Erfassen (530) des Lichts, das den thermochromen Teil passiert hat; und
Bestimmen (540) einer Körpertemperatur des Objekts durch Verwenden des Spektrums des erfassten Lichts, wobei das Bestimmen umfasst:
Schätzen einer Position eines Blutgefäßes (VE) auf der Basis des Spektrums des erfassten Lichts, und
Schätzen einer ersten Körpertemperatur an der geschätzten Position des Blutgefäßes, Schätzen einer zweiten Körpertemperatur an einer anderen Position als der Position des Blutgefäßes und Bestimmen der Körpertemperatur des Objekts auf der Basis der geschätzten ersten Körpertemperatur und der geschätzten zweiten Körpertemperatur.

2. Verfahren (500) nach Anspruch 1, wobei das Bestimmen (540) das Bestimmen der Körpertemperatur des Objekts (OBJ) durch Vergleichen eines Referenzspektrums mit dem Spektrum umfasst, das sich entsprechend der thermochromen Eigenschaft des thermochromen Teils (112; 712) geändert hat, und
wobei das Bestimmen das Bestimmen eines Lichtspektrums, das wenigstens einen Abschnitt (21) des thermochromen Teils, der keine thermochrome Eigenschaft aufweist, passiert hat, als das Referenzspektrum umfasst.

3. Verfahren (500) nach Anspruch 1 oder 2, ferner umfassend:
Schätzen einer ersten Körpertemperatur auf der Basis eines Spektrums, das durch eine erste thermochrome Schicht (112b) des thermochromen Teils (112; 712) verändert wird;
Schätzen einer zweiten Körpertemperatur auf der Basis eines Spektrums, das von einer zweiten thermochromen Schicht (112c) des thermochromen Teils verändert wird; und
Schätzen eines Wärmeflusses auf der Basis der geschätzten ersten Körpertemperatur, der geschätzten zweiten Körpertemperatur und einer Wärmeleitfähigkeit eines wärmeleitenden Materials (112a) des thermochromen Teils, und
wobei das Bestimmen (540) das Bestimmen der Körpertemperatur des Objekts (OBJ) auf der Basis der geschätzten ersten Körpertemperatur, der geschätzten zweiten Körpertemperatur und des geschätzten Wärmeflusses umfasst.

4. Vorrichtung (100; 700a; 700b; 900; 1000) zum Schätzen von Bioinformationen, wobei die Vorrichtung umfasst:
einen ersten Sensor (110; 710), umfassend:
einen ersten Lichtquellenteil (111; 711), ausgebildet zum Ausstrahlen (510) eines ersten Lichts auf ein Objekt (OBJ); einen ersten thermochromen Teil (112; 712), ausgebildet zum Ändern (520) eines Spektrums des ersten Lichts, das vom Objekt reflektiert oder gestreut wird und dieses passiert, entsprechend einer thermochromen Eigenschaft des ersten thermochromen Teils; und
einen ersten Lichtempfänger (113; 713), ausgebildet zum Erfassen (530) des ersten Lichts, das den ersten thermochromen Teil passiert hat; und
einen Prozessor (120; 720), ausgebildet zum Bestimmen (540) einer Körpertemperatur des Objekts auf der Basis des Spektrums des vom ersten Sensor erfassten ersten Lichts durch Schätzen einer Position eines Blutgefäßes (VE) auf der Basis des Spektrums des erfassten Lichts, Schätzen einer ersten Körpertemperatur an der geschätzten Position des Blutgefäßes, Schätzen einer zweiten Körpertemperatur an einer anderen Position als der Position des Blutgefäßes und Bestimmen der Körpertemperatur des Objekts auf der Basis der geschätzten ersten Körpertemperatur und der geschätzten zweiten Körpertemperatur.

5. Vorrichtung (100; 700a; 700b; 900; 1000) nach Anspruch 4, wobei die Vorrichtung wenigstens ein Element der Gruppe umfassend eine Smart Watch, ein intelligentes Armband, eine intelligente Brille, einen intelligenten Ohrhörer, eine intelligente Halskette, ein Smartphone und einen Tablet-Personalcomputer(-PC) ist.

6. Vorrichtung (100; 700a; 700b; 900; 1000) nach Anspruch 5, ferner umfassend einen Kraft-/ Drucksensor (760), ausgebildet zum Erhalten einer Kontaktkraft und/oder eines Kontaktdrucks zwischen dem ersten Sensor (110; 710) und dem Objekt (OBJ), das mit dem ersten Sensor in Kontakt kommt, ermittelt, und wobei der Prozessor (120; 720) ferner ausgebildet ist zum Erzeugen eines Oszillogramms auf der Basis eines Pulswellensignals und der Kontaktkraft und/oder des Kontaktdrucks und Schätzen von Bioinformationen auf der Basis des erzeugten Oszillogramms, wobei das Pulswellensignal vom Prozessor auf der Basis einer Intensität des ersten Lichts extrahiert wird, das für einen vorbestimmten Zeitraum erfasst wird.

7. Vorrichtung (100; 700a; 700b; 900; 1000) nach Anspruch 5 oder 6, ferner umfassend einen zweiten Sensor (1010), wobei der zweite Sensor einen zweiten Lichtquellenteil, ausgebildet zum Ausstrahlen eines zweiten Lichts auf das Objekt (OBJ), einen zweiten thermochromen Teil, ausgebildet zum Ändern eines Spektrums des zweiten Lichts, das vom Objekt reflektiert oder gestreut wird und dieses passiert, entsprechend einer thermochromen Eigenschaft des zweiten thermochromen Teils, und einen zweiten Lichtempfänger, ausgebildet zum Erfassen des zweiten Lichts, das den zweiten thermochromen Teil passiert hat, umfasst.

8. Vorrichtung (100; 700a; 700b; 900; 1000) nach Anspruch 7, wobei der Prozessor (120; 720) ferner ausgebildet ist zum Schätzen der Bioinformationen auf der Basis eines ersten Pulswellensignals, das erhalten wird, wenn ein erstes Objekt mit dem ersten Sensor in Kontakt kommt, und eines zweiten Pulswellensignals, das erhalten wird, wenn ein zweites Objekt mit dem zweiten Sensor in Kontakt kommt.

9. Vorrichtung (100; 700a; 700b; 900; 1000) nach Anspruch 8, wobei der Prozessor (120; 720) ferner ausgebildet ist zum Erhalten einer Pulse Transit Time (PTT) auf der Basis des ersten Pulswellensignals und des zweiten Pulswellensignals und Schätzen der Bioinformationen auf der Basis der erhaltenen PTT, oder
wobei der Prozessor ferner ausgebildet ist zum Erhalten eines dritten Pulswellensignals auf der Basis des ersten Pulswellensignals und des zweiten Pulswellensignals und Schätzen der Bioinformationen auf der Basis des erhaltenen dritten Pulswellensignals unter Verwendung von Oszillometrie.

10. Vorrichtung (100; 700a; 700b; 900; 1000) nach Anspruch 7, wobei:
der erste Sensor (110; 710) auf einer ersten Fläche eines Hauptkörpers der Vorrichtung angeordnet ist, die in Kontakt mit dem Objekt (OBJ) kommt; und
der zweite Sensor (1010) auf einer zweiten Fläche des Hauptkörpers angeordnet ist, wobei die zweite Fläche zu einer Außenseite freiliegt, wenn das Objekt mit dem ersten Sensor in Kontakt ist.

11. Vorrichtung (100; 700a; 700b; 900; 1000) nach Anspruch 10, wobei der Prozessor (120; 720) ferner ausgebildet ist zum Bestimmen der Körpertemperatur des Objekts (OBJ) auf der Basis des vom ersten Lichtempfänger (113; 713) erfassten Spektrums des ersten Lichts, Bestimmen einer Außentemperatur auf der Basis des vom zweiten Lichtempfänger erfassten Spektrums des zweiten Lichts und Korrigieren der bestimmten Körpertemperatur des Objekts auf der Basis der erhaltenen Außentemperatur.

12. Vorrichtung (100; 700a; 700b; 900; 1000) nach Anspruch 7, wobei der zweite Lichtquellenteil des zweiten Sensors (1010) auf einem Außenlicht der Vorrichtung basiert.

13. Vorrichtung (100; 700a; 700b; 900; 1000) nach Anspruch 5, ferner umfassend einen Temperatursensor, der auf einer Fläche eines Hauptkörpers der Vorrichtung angeordnet ist, wobei die Fläche zu einer Außenseite freiliegt, wenn das Objekt in Kontakt mit dem ersten Sensor ist,
wobei der Prozessor (120; 720) ferner ausgebildet ist zum Bestimmen der Körpertemperatur des Objekts (OBJ) auf der Basis des vom ersten Lichtempfänger (113; 713) erfassten Spektrums des ersten Lichts und Korrigieren der bestimmten Körpertemperatur des Objekts auf der Basis einer vom Temperatursensor erhaltenen Außentemperatur.

14. Vorrichtung (100; 700a; 700b; 900; 1000) nach Anspruch 6, wobei die Bioinformationen wenigstens ein Element umfasst der Gruppe umfassend Triglycerid, Körperfettanteil, Körperwasser, Blutglukose, Cholesterin, Carotinoid, Protein, Harnsäure, Blutdruck, Gefäßalter, arterielle Steifigkeit, Wellenform des Aortendrucks, Gefäßcompliance, Stressindex, Ermüdungsgrad, Hautalter und Hautelastizität.

## Revendications

1. Procédé (500) de mesure d'une température corporelle, le procédé comprenant :
l'émission (510) d'une lumière sur un objet (OBJ) par une partie source de lumière (111 ; 711) ;
la modification (520), en utilisant une partie thermochromique (112 ; 712), d'un spectre de la lumière, qui est réfléchie ou diffusée par l'objet et le traverse, selon une propriété thermochromique de la partie thermochromique ;
la détection (530) de la lumière ayant traversé la partie thermochromique ; et
la détermination (540) d'une température corporelle de l'objet en utilisant le spectre de la lumière détectée, dans lequel la détermination comprend :
l'estimation d'une position d'un vaisseau sanguin (VE) sur la base du spectre de la lumière détectée, et
l'estimation d'une première température corporelle à la position estimée du vaisseau sanguin, l'estimation d'une seconde température corporelle à une position autre que la position du vaisseau sanguin, et la détermination de la température corporelle de l'objet sur la base de la première température corporelle estimée et de la seconde température corporelle estimée.

2. Procédé (500) selon la revendication 1, dans lequel la détermination (540) comprend la détermination de la température corporelle de l'objet (OBJ) en comparant un spectre de référence avec le spectre ayant été modifié en fonction de la propriété thermochromique de la partie thermochromique (112 ; 712), et
dans lequel la détermination comprend la détermination d'un spectre de lumière ayant traversé au moins une portion (21) de la partie thermochromique ne présentant pas de propriété thermochromique, comme étant le spectre de référence.

3. Procédé (500) selon la revendication 1 ou 2, comprenant en outre :
l'estimation d'une première température corporelle sur la base d'un spectre modifié par une première couche thermochromique (112b) de la partie thermochromique (112 ; 712) ;
l'estimation d'une seconde température corporelle sur la base d'un spectre modifié par une seconde couche thermochromique (112c) de la partie thermochromique ; et
l'estimation d'un flux de chaleur sur la base de la première température corporelle estimée, de la seconde température corporelle estimée, et de la conductivité thermique d'un matériau thermiquement conducteur (112a) de la partie thermochromique, et
dans lequel la détermination (540) comprend la détermination de la température corporelle de l'objet (OBJ) sur la base de la première température corporelle estimée, de la seconde température corporelle estimée et du flux de chaleur estimé.

4. Appareil (100 ; 700a ; 700b ; 900 ; 1000) d'estimation d'informations biologiques, l'appareil comprenant :
un premier capteur (110 : 710) comprenant :
une première partie source de lumière (111 ; 711) configurée pour émettre (510) une première lumière vers un objet (OBJ) ;
une première partie thermochromique (112 ; 712), configurée pour modifier (520) un spectre de la première lumière, laquelle est réfléchie ou diffusée par l'objet et le traverse, en fonction d'une propriété thermochromique de la première partie thermochromique ; et
un premier récepteur de lumière (113 ; 713) configuré pour détecter (530) la première lumière ayant traversé la première partie thermochromique ; et
un processeur (120 ; 720) configuré pour déterminer (540) une température corporelle de l'objet sur la base du spectre de la première lumière détectée par le premier capteur, en estimant une position d'un vaisseau sanguin (VE) sur la base du spectre de la lumière détectée, en estimant une première température corporelle à la position estimée du vaisseau sanguin, en estimant une seconde température corporelle à une position autre que la position du vaisseau sanguin, et en déterminant la température corporelle de l'objet sur la base de la première température corporelle estimées et de la seconde température corporelle estimée.

5. Appareil (100 ;700a ; 700b ; 900 ; 1000) selon la revendication 4, dans lequel l'appareil est au moins l'un parmi une montre connectée, un bracelet connecté, des lunettes connectées, des écouteurs intelligents, un collier intelligent, un smartphone ou un ordinateur personnel de type tablette, PC.

6. Appareil (100 ; 700a ; 700b ; 900 ; 1000) selon la revendication 5, comprenant en outre un capteur de force/de pression (760) configuré pour obtenir une force de contact et/ou une pression de contact entre le premier capteur (110 ; 710) et l'objet (OBJ) qui entre en contact avec le premier capteur, et dans lequel le processeur (120 ; 720) est en outre configuré pour générer un oscillogramme sur la base d'un signal d'onde de pouls et de la force de contact et/ou de la pression de contact, et estimer des informations biologiques sur la base de l'oscillogramme généré, dans lequel le signal d'onde de pouls est extrait par le processeur sur la base de l'intensité de la première lumière qui est détectée pendant une période de temps prédéterminée.

7. Appareil (100 ; 700a ; 700b ; 900 ; 1000) selon la revendication 5 ou 6, comprenant en outre un second capteur (1010), le second capteur comprenant une seconde partie source de lumière configurée pour émettre une seconde lumière vers l'objet (OBJ), une seconde partie thermochromique configurée pour modifier un spectre de la seconde lumière, qui est réfléchie ou diffusée par l'objet et le traverse, selon une propriété thermochromique de la seconde partie thermochromique, et un second récepteur de lumière configuré pour détecter la seconde lumière ayant traversé la seconde partie thermochromique.

8. Appareil (100 ; 700a ; 700b ; 900 ; 1000) selon la revendication 7, dans lequel le processeur (120 ; 720) est en outre configuré pour estimer les informations biologiques sur la base d'un premier signal d'onde de pouls obtenu lorsqu'un premier objet entre en contact avec le premier capteur, et d'un deuxième signal d'onde de pouls obtenu lorsqu'un second objet entre en contact avec le second capteur.

9. Appareil (100 ; 700a ; 700b ; 900 ; 1000) selon la revendication 8, dans lequel le processeur (120 ; 720) est en outre configuré pour obtenir un temps de transit du pouls, PTT, sur la base du premier signal d'onde de pouls et du deuxième signal d'onde de pouls, et estimer les informations biologiques sur la base du PTT obtenu, ou
dans lequel le processeur est en outre configuré pour obtenir un troisième signal d'onde de pouls sur la base du premier signal d'onde de pouls et du deuxième signal d'onde de pouls, et estimer les informations biologiques sur la base du troisième signal d'onde de pouls obtenu, en utilisant l'oscillométrie.

10. Appareil (100 ; 700a ; 700b ; 900 ; 1000) selon la revendication 7, dans lequel :
le premier capteur (110 ; 710) est disposé sur une première surface d'un corps principal de l'appareil qui entre en contact avec l'objet (OBJ) ; et
le second capteur (1010) est disposé sur une seconde surface du corps principal, la seconde surface étant exposée vers l'extérieur lorsque l'objet est en contact avec le premier capteur.

11. Appareil (100 ; 700a ; 700b ; 900 ; 1000) selon la revendication 10, dans lequel le processeur (120 ; 720) est en outre configuré pour déterminer la température corporelle de l'objet (OBJ) sur la base du spectre de la première lumière détectée par le premier récepteur de lumière (113 ; 713), obtenir une température externe sur la base du spectre de la seconde lumière détectée par le second récepteur de lumière, et corriger la température corporelle déterminée de l'objet sur la base de la température externe obtenue.

12. Appareil (100 ; 700a ; 700b ; 900 ; 1000) selon la revendication 7, dans lequel la seconde source de lumière du second capteur (1010) est basée sur une lumière externe de l'appareil.

13. Appareil (100 ; 700a ; 700b ; 900 ; 1000) selon la revendication 5, comprenant en outre un capteur de température disposé sur une surface d'un corps principal de l'appareil, la surface étant exposée vers l'extérieur lorsque l'objet est en contact avec le premier capteur,
dans lequel le processeur (120 ; 720) est en outre configuré pour déterminer la température corporelle de l'objet (OBJ) sur la base du spectre de la première lumière détectée par le premier récepteur de lumière (113 ; 713), et corriger la température corporelle déterminée de l'objet sur la base d'une température externe obtenue par le capteur de température.

14. Appareil (100 ; 700a ; 700b ; 900 ; 1000) selon la revendication 6, dans lequel les informations biologiques comprennent au moins l'un parmi le triglycéride, le pourcentage de masse graisseuse, l'eau corporelle, la glycémie, le cholestérol, le caroténoïde, la protéine, l'acide urique, la pression artérielle, l'âge vasculaire, la rigidité artérielle, la courbe de pression aortique, la compliance vasculaire, l'indice de stress, le niveau de fatigue, l'âge de la peau ou l'élasticité de la peau.
